(19) Europäisches Patentamt
European Patent Office
Office européen des brevets

(11) Veröffentlichungsnummer: **0 035 641**
**B1**

(12) # EUROPÄISCHE PATENTSCHRIFT

(45) Veröffentlichungstag der Patentschrift: **25.04.84**

(21) Anmeldenummer: **81100677.4**

(22) Anmeldetag: **30.01.81**

(51) Int. Cl.³: **C 07 C 149/247,** C 07 D 307/79, A 61 K 31/34, A 61 K 31/195, A 61 K 31/22

(54) **Cysteinderivate, Verfahren zur Herstellung derselben und diese enthaltende Arzneimittel.**

(30) Priorität: **31.01.80 IT 332680**

(43) Veröffentlichungstag der Anmeldung: **16.09.81 Patentblatt 81/37**

(45) Bekanntmachung des Hinweises auf die Patenterteilung: **25.04.84 Patentblatt 84/17**

(84) Benannte Vertragsstaaten: **BE DE FR GB NL**

(56) Entgegenhaltungen:
**DE - A - 1 793 828**
**DE - A - 2 526 089**
**GB - A - 954 268**

**Die Akte enthält technische Angaben, die nach dem Eingang der Anmeldung eingereicht wurden und die nicht in dieser Patentschrift enthalten sind.**

(73) Patentinhaber: **ALFA FARMACEUTICI S.p.A., Via Ragazzi del '99 no 5, I-40133 Bologna (IT)**

(72) Erfinder: **Mascellani, Giuseppe, Via Lavino No 273/9, Calderino di Monte San Pietro (BO) (IT)**
Erfinder: **Tamagnone, Gianfranco, Via Ronzani no 55, Casalecchio di Reno (BO) (IT)**
Erfinder: **Guerra, Guido, Via Irma Bandiera no 11, I-40100 Bologna (IT)**
Erfinder: **Benelli, Augusta, Via Beraldi no 28, I-41100 Modena (IT)**

(74) Vertreter: **Beszédes, Stephan G. Dr., Münchener Strasse 80a Postfach 1168, D-8060 Dachau (DE)**

**EP 0 035 641 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Cysteinderivate, Verfahren zur Herstellung derselben und diese enthaltende Arzneimittel

Die Erfindung betrifft neue Cysteinderivate, ein Verfahren zur Herstellung derselben sowie diese Verbindungen enthaltende Arzneimittel, insbesondere solche mit entzündungshemmenden, schmerzlindernden beziehungsweise analgesischen, fiebersenkenden beziehungsweise antipyretischen und schleimlösenden beziehungsweise mucolytischen Wirkungen.

Aus dem Schrifttum sind mehrere Cysteinderivate bekannt. Beispielsweise sind zur Behandlung der Arteriosklerose brauchbare funktionelle L-Cysteinderivate in den deutschen Offenlegungsschriften 2 329 819 und 2 329 821 beschrieben. Aus der deutschen Offenlegungsschrift 2 027 832 ist ein Cysteinderivat mit antibakterieller Wirksamkeit, welches durch Umsetzen von N-Acetylcystein mit dem Glykollester von Thiamphenikol hergestellt wird, bekannt. Ähnlich sind in der japanischen Patentanmeldung 7 435 334 gegen C. lagenarium und P. citrinum wirksame andere Cysteinderivate beschrieben. Schließlich betrifft die französische Offenlegungsschrift 2 147 812 Halbsalze von S-Carboxymethylcystein mit Alkali- oder Erdalkalimetallen oder mit Stickstoff aufweisenden Verbindungen, wie Äthanolamin, Piperazin, Piperidin, Lysin, Arginin oder Histidin. Für diese Verbindungen wurden pharmakologische Eigenschaften und auch die Verwendung in kosmetischen Präparaten angegeben.

Ferner sind aus der britischen Patentschrift 954 268 N-Acylcysteinderivate, nämlich N-Alkanoyl- und N-Benzoylcysteinderivate, bei welchen an das Schwefelatom zwingend ein Wasserstoffatom gebunden ist, bekannt. Auch ist deren mucolytische Wirkung erwähnt, während von anderen pharmakologischen Wirkungen keine Rede ist. Auf Grund von eigenen Versuchen haben die Verbindungen dieser Druckschrift keine entzündungshemmende Wirkung.

Weiterhin ist in der deutschen Offenlegungsschrift 2 526 089 unter anderem die Verbindung 2-Äthyl-2,3-dihydrobenzofuran-5-ylessigsäure beschrieben. Keine der Verbindungen dieser Druckschrift hat jedoch einen Cysteinrest. Auch ist in der genannten Druckschrift die entzündungshemmende und schmerzlindernde Wirkung von deren Verbindungen beschrieben.

Außerdem sind aus der deutschen Offenlegungsschrift 1 793 828 2-(6'-Methoxy-2'-naphthyl)-propionsäure und Amide derselben bekannt. Als pharmakologische Wirkungen dieser Verbindungen sind in der genannten Druckschrift die entzündungshemmende, analgetische, antipyretische und antipruritische Wirkung angegeben. In Wirklichkeit ist jedoch die entzündungshemmende Wirkung dieser Verbindungen nicht zufriedenstellend.

Der Erfindung liegt die Aufgabe zugrunde, neue Cysteinderivate mit überlegenen pharmakologischen, insbesondere entzündungshemmenden, schmerzlindernden, fiebersenkenden und schleimlösenden, Wirkungen, ein Verfahren zur Herstellung derselben und diese Verbindungen enthaltende Arzneimittel vorzusehen.

Das Obige wurde überraschenderweise durch die Erfindung erreicht.

Gegenstand der Erfindung sind Cysteinderivate der allgemeinen Formel

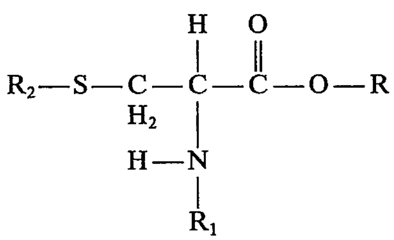

(I)

worin

- R für Wasserstoff, einen Alkylrest mit 1 bis 4 Kohlenstoffatom(en), ein Alkali- oder Erdalkalimetall-Äquivalent oder eine quaternäre Ammoniumgruppe steht,
- $R_1$ einen Alkylcarbonylrest (Alkanoylrest) mit 2 bis 4 Kohlenstoffatomen, einen 2-(6'-Methoxynaphth-2'-yl)-propionylrest oder einen 2-(2'-Äthyl-2',3'-dihydrobenzofuran-5'-yl)-acetylrest bedeutet und
- $R_2$ Wasserstoff, einen 2-(6'-Methoxynaphth-2'-yl)-propionylrest oder einen 2-(2'-Äthyl-2',3'-dihydrobenzofuran-5'-yl)-acetylrest darstellt,

mit den weiteren Maßgaben, daß

a) im Falle, daß

eines von $R_1$ und $R_2$ für einen 2-(6'-Methoxynaphth-2'-yl)-propionylrest oder einen 2-(2'-Äthyl-2',3'-dihydrobenzofuran-5'-yl)-acetylrest steht,
das andere von $R_1$ und $R_2$ von einem 2-(6'-Methoxynaphth-2'-yl)-propionylrest und 2-(2'-Äthyl-2',3'-dihydrobenzofuran-5'-yl)-acetylrest verschieden ist, und

b) im Falle, daß

$R_1$ für einen Alkylcarbonylrest mit 2 bis 4 Kohlenstoffatomen steht,
$R_2$ von Wasserstoff verschieden ist,

in Form der getrennten optisch aktiven Isomere oder von Mischungen derselben.

Vorzugsweise ist der Alkylrest, für den R stehen kann, ein solcher mit 1 oder 2, insbesondere 1, Kohlenstoffatom(en).

Es ist auch bevorzugt, daß der Alkylcarbonylrest, für den $R_1$ stehen kann, ein solcher mit 2 oder 3, insbesondere 2, Kohlenstoffatomen ist.

Eine besonders bevorzugte erfindungsgemäße Verbindung ist N-Acetyl-S-[2-(6'-methoxynaphth-2'-yl)-propionyl]-cystein.

Bevorzugte Alkalimetalle, für welche R stehen kann, sind Natrium und Kalium.

Vorzugsweise ist das Erdalkalimetall, für welches R stehen kann, Calcium.

Es ist auch bevorzugt, daß die quaternäre Ammoniumgruppe, für welche R stehen kann, eine Tetraalkylammoniumgruppe, deren Alkylgruppen nicht mehr als 4 Kohlenstoffatome aufweisen, ist.

Als optisch aktive Isomere der erfindungsgemäßen Cysteinderivate der allgemeinen Formel I sind diejenigen, welche den L-Cysteinmolekülrest aufweisen, bevorzugt.

Im Falle des Vorliegens eines 2-(6'-Methoxynaphth-2'-yl)-propionylrestes hat dieser vorzugsweise die D-Konfiguration.

Im Gegensatz zu den Verbindungen der britischen Patentschrift 954 268 ist bei den erfindungsgemäßen Verbindungen, im Falle daß an das Stickstoffatom (als $R_1$) ein Alkylcarbonylrest mit 2 bis 4 Kohlenstoffatomen gebunden ist, an das Schwefelatom zwingend kein Wasserstoffatom gebunden ($R_2$ ist von Wasserstoff verschieden), wie es oben in der weiteren Maßgabe b) festgelegt ist.

Von sämtlichen Verbindungen der deutschen Offenlegungsschrift 2 526 089 unterscheiden sich die erfindungsgemäßen Verbindungen darin, daß sie einen Cysteinrest aufweisen.

Auch von den Verbindungen der deutschen Offenlegungsschrift 1 793 828 unterscheiden sich die erfindungsgemäßen Verbindungen darin, daß sie einen Cysteinrest aufweisen. Nach dazu ist in den erfindungsgemäßen Verbindungen, welche keine Amide sind, das Stickstoffatom durch ganz andere Reste substituiert als das Amidstickstoffatom der Verbindungen der deutschen Offenlegungsschrift 1 793 828. Die erfindungsgemäßen Verbindungen sind denen der deutschen Offenlegungsschrift 1 793 828 insbesondere hinsichtlich der entzündungshemmenden Wirkung überlegen.

Die erfindungsgemäßen Verbindungen können nach bekannten Verfahrensweisen zum Acylieren vom Aminen und Thioalkoholen je nach den Ausgangsmaterialien und gewünschten Endprodukten hergestellt werden.

Gegenstand der Erfindung ist daher auch ein Verfahren zur Herstellung der erfindungsgemäßen Verbindungen, welches dadurch gekennzeichnet ist, daß in an sich bekannter Weise

a) 1 Moläquivalent Cysteinderivate der allgemeinen Formel

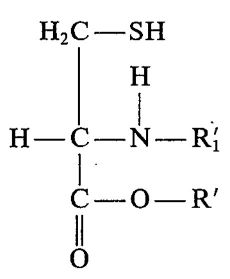

(II)

worin

R' für Wasserstoff oder einen Alkylrest mit 1 bis 4 Kohlenstoffatomen steht und
$R'_1$ Wasserstoff oder einen Alkylcarbonylrest mit 2 bis 4 Kohlenstoffatomen bedeutet,

mit etwa 1 Moläquivalent oder einem geringen Überschuß von 2-(6'-Methoxynaphth-2'-yl)-propionyl- oder 2-(2'-Äthyl-2',3'-dihydrobenzofuran-5'-yl)-acetylhalogeniden in organischen Lösungsmitteln in Gegenwart von mindestens 1 Moläquivalent alkalischen Mitteln, bezogen auf das Cysteinderivat der allgemeinen Formel II, gegebenenfalls in einer Stickstoffatmosphäre, umgesetzt wird oder

b) zur Herstellung der Verbindungen der allgemeinen Formel I, bei welchen

$R_1$ für einen 2-(6'-Methoxynaphth-2'-yl)-propionyl- oder 2-(2'-Äthyl-2',3'-dihydrobenzofuran-5'-yl)-acetylrest steht und
$R_2$ Wasserstoff bedeutet,

1 Moläquivalent Cysteinderivate der allgemeinen Formel

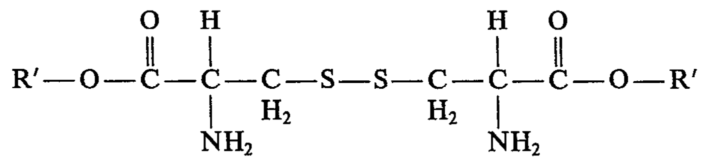

(III)

worin R′ für Wasserstoff oder einen Alkylrest mit 1 bis 4 Kohlenstoffatomen steht, oder Säureadditionssalze derselben mit etwa 2 Moläquivalenten oder einem geringen Überschuß darüber von 2-(6′-Methoxynaphth-2′-yl)-propionyl- oder 2-(2′-Äthyl-2′,3′-dihydrobenzofuran-5′-yl)-acetylhalogeniden in inerten organischen Lösungsmitteln in Gegenwart von alkalischen Mitteln zu $N^1$,$N^2$-bis-[2-(6′-Methoxynaphth-2′-yl)-propionyl]-cystin beziehungsweise $N^1$,$N^2$-bis-[2-(2′-Äthyl-2′,3′-dihydrobenzofuran-5′-yl)-acetyl]-cystin beziehungsweise ihren Estern der allgemeinen Formel

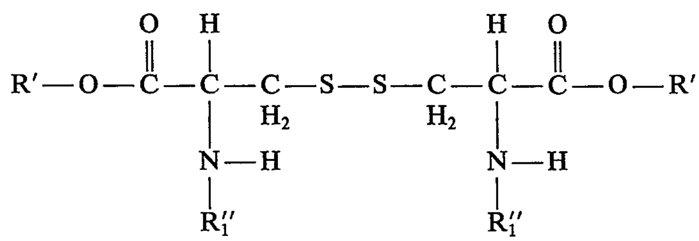

(IV)

worin

$R_1''$ für einen 2-(6′-Methoxynaphth-2′-yl)-propionyl- oder 2-(2′-Äthyl-2′,3′-dihydrobenzofuran-5′-yl)-acetylrest steht und
R′ wie vorstehend festgelegt ist,

umgesetzt wird und die letzteren, gegebenenfalls ohne ihre Isolierung, katalytisch hydriert werden oder c) zur Herstellung der Verbindungen der allgemeinen Formel I, bei welchen

R für Wasserstoff, ein Alkali- oder Erdalkalimetall-Äquivalent oder eine quaternäre Ammoniumgruppe steht,
$R_1$ einen Alkylcarbonylrest mit 2 bis 4 Kohlenstoffatomen bedeutet und
$R_2$ einen 2-(6′-Methoxynaphth-2′-yl)-propionyl- oder 2-(2′-Äthyl-2′,3′-dihydrobenzofuran-5′-yl)-acetylrest darstellt,

1 Moläquivalent Cysteinderivate der allgemeinen Formel

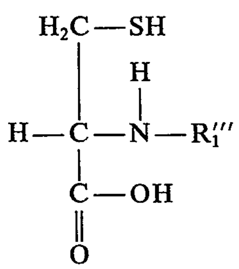

(V)

worin $R_1'''$ für einen Alkylcarbonylrest mit 2 bis 4 Kohlenstoffatomen steht, beziehungsweise Säureadditionssalze derselben, mit etwa 1 bis 3 Moläquivalenten Trialkylsilylhalogeniden der allgemeinen Formel

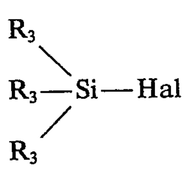

(VI)

worin

$R_3$ für einen Alkylrest mit 1 bis 4 Kohlenstoffatomen steht und
Hal Halogen bedeutet,

in inerten organischen Lösungsmitteln in Gegenwart von mindestens 2 Moläquivalenten von tertiären Stickstoff aufweisenden organischen Basen zu geschützten Cysteinderivaten (Trialkylsilylcysteinderi-

vaten) der allgemeinen Formel

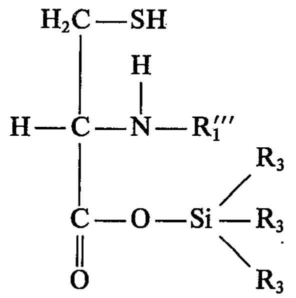

(VII)

worin $R_1'''$ und $R_3$ wie vorstehend festgelegt sind, umgesetzt wird, diese durch Zugabe von etwa 1 Moläquivalent, vorzugsweise 0,9 bis 1,1 Moläquivalent, 2-(6'-Methoxynaphth-2'-yl)-propionyl- beziehungsweise 2-(2'- Äthyl-2',3'-dihydrobenzofuran-5'-yl)-acetylhalogeniden zu geschützten Cysteinderivaten der allgemeinen Formel

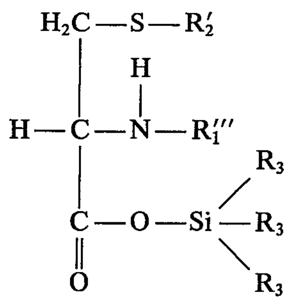

(VIII)

worin

$R_2'$ für einen 2-(6'-Methoxynaphth-2'-yl)-propionyl- oder 2-(2'-Äthyl-2',3'-dihydrobenzofuran-5'- yl)-acetylrest steht und
$R_1'''$ und $R_3$ wie vorstehend festgelegt sind,

umgesetzt werden und die die letzteren enthaltenden Reaktionsmischungen zur Wiederherstellung der freien Carboxylgruppe unter Entfernung der Trialkylsilylgruppe mit Wasser behandelt werden, wobei alle diese Umsetzungen ohne Isolierung von Zwischenprodukten durchgeführt werden können, worauf in an sich bekannter Weise gegebenenfalls die gegebenenfalls erhaltenen Cysteinderivate der allgemeinen Formel I, bei welchem R für Wasserstoff steht, in ihre Alkali- oder Erdalkalimetallsalze oder quaternären Ammoniumsalze überführt werden und/oder gegebenenfalls die erhaltenen Mischungen der optisch aktiven Isomere der Cysteinderivate der allgemeinen Formel I in die optisch aktiven Isomere gespalten beziehungsweise die erhaltenen optisch aktiven Isomere der Cysteinderivate der allgemeinen Formel I racemisiert werden.

Vorzugsweise wird die Umsetzung der Cysteinderivate der allgemeinen Formel II mit den 2-(6'-Methoxynaphth-2'-yl)-propionylhalogeniden beziehungsweise 2-(2'-Äthyl-2',3'-dihydrobenzofuran-5'-yl)-acetylhalogeniden der Variante a) des erfindungsgemäßen Verfahrens bei einer Temperatur von etwa −10° C bis Raumtemperatur durchgeführt. Auch ist für diese Umsetzung eine Reaktionsdauer von etwa 4 bis 7 Stunden bevorzugt.

Vorteilhaft können als organische Lösungsmittel für die genannte Umsetzung Tetrahydrofuran, Dioxan, Wasser, Dimethylformamid, Äthylacetat oder niedere Halogenkohlenwasserstoffe oder Mischungen derselben verwendet werden.

Zweckmäßig kann die Variante a) des erfindungsgemäßen Verfahrens wie folgt durchgeführt werden. Die genannten beiden Reaktionsteilnehmer werden in äquimolaren Mengen in einem organischen Lösungsmittel zusammengebracht; manchmal ist aber die Verwendung eines geringen, vorzugsweise bis zu 1,5-fach molaren, Überschusses des 2-(6'-Methoxynaphth-2'-yl)-propionyl- beziehungsweise 2-(2'-Äthyl-2',3'-dihydrobenzofuran-5'-yl)-acetylhalogenides vorteilhaft. Zum Reaktionsmedium wird auch mindestens 1 Moläquivalent eines alkalischen Mittels, bezogen auf das Ausgangscysteinderivat der allgemeinen Formel II, deswegen zugegeben, um die sich während des Reaktionsverlaufes bildende Acidität zu binden. Vorteilhaft können als alkalische Mittel organische tertiäre Stickstoff aufweisende Basen, wie Pyridin, Picoline oder Triäthylamin, oder wäßrige Alkalien, wie Natriumhydroxyd, Kaliumhydroxyd, Natriumcarbonat, Kaliumcarbonat, Natriumacetat oder Kaliumacetat, verwendet werden.

Manchmal ist es zweckmäßig, in einer Stickstoffatmosphäre zu arbeiten, um durch Entfernung des Sauerstoffes von der Reaktionsumgebung die Oxydation des Cysteinderivates zu vermeiden.

Je nach dem verwendeten Cysteinderivat der allgemeinen Formel II werden durch die genannte Umsetzung Cysteinderivatendprodukte der allgemeinen Formel I, bei welchen R für Wasserstoff oder einen Alkylrest mit 1 bis 4 Kohlenstoffatomen steht, $R_1$ einen 2-(6'-Methoxynaphth-2'-yl)-propionylrest oder 2-(2'-Äthyl-2',3'-dihydrobenzofuran-5'-yl)-acetylrest bedeutet und $R_2$ Wasserstoff darstellt, oder Cysteinderivatendprodukte der allgemeinen Formel I, bei welchen R wie vorstehend festgelegt ist, $R_1$ für einen Alkylcarbonylrest mit 2 bis 4 Kohlenstoffatomen steht und $R_2$ einen 2-(6'-Methoxynaphth-2'-yl)-propionylrest oder 2-(2'-Äthyl-2',3'-dihydrobenzofuran-5'-yl)-acetylrest bedeutet, erhalten.

Bei der Umsetzung der Cysteinderivate der allgemeinen Formel III mit den 2-(6'-Methoxynaphth-2'-yl)-propionylhalogeniden beziehungsweise 2-(2'-Äthyl-2',3'-dihydrobenzofuran-5'-yl)-acetylhalogeniden der Variante b) des erfindungsgemäßen Verfahrens wird bevorzugt ein leichter Überschuß der letzteren über die Menge von 2 Mol, vorzugsweise bis zu 2,5 Mol, insbesondere bis zu 2,2 Mol, je Mol der ersteren verwendet.

Vorzugsweise wird diese Umsetzung bei Temperaturen von etwa 4° C bis Raumtemperatur durchgeführt. Auch ist für diese Umsetzung eine Reaktionsdauer von etwa 4 bis 7 Stunden bevorzugt.

Vorteilhaft werden als inerte organische Lösungsmittel für diese Umsetzung Benzol, Toluol oder niedere Halogenkohlenwasserstoffe oder Mischungen derselben verwendet.

Zweckmäßig wird die Variante b) des erfindungsgemäßen Verfahrens wie folgt durchgeführt.

Es wird in heterogener Phase gearbeitet und als alkalische Mittel werden vorteilhaft Natrium- oder Kaliumhydroxydlösungen verschiedener Konzentrationen oder wäßrige Lösungen von Carbonaten dieser Alkalimetalle verwendet. Gegebenenfalls können die Zwischenprodukte $N^1$,$N^2$-bis-[2-(6'-Methoxynaphth-2'-yl)-propionyl]-cystin- beziehungsweise $N^1$,$N^2$-bis-[2-(2'-Äthyl-2',3'-dihydrobenzofuran-5'-yl)-acetyl]-cystin beziehungsweise deren Ester der allgemeinen Formel IV, welche Verbindungen also an beiden Stickstoffatomen je einen 2-(6'-Methoxynaphth-2'-yl)-propionylrest oder 2-(2'-Äthyl-2',3'-dihydrobenzofuran-5'-yl)-acetylrest aufweisen, isoliert werden, wie bereits erwähnt können jedoch diese Zwischenprodukte auch, so wie sie angefallen sind, für die anschließende Reaktionsstufe, bei welcher die Bindung —S—S— hydriert wird und die gewünschten Cysteinderivatendprodukte der allgemeinen Formel I erhalten werden, eingesetzt werden. Die katalytische Hydrogenolyse beziehungsweise Hydrierung kann in Gegenwart von üblicherweise für solche Umsetzungen verwendeten Katalysatoren, beispielsweise verschiedenen Arten von Raney-Nickel, durchgeführt werden, es wurde jedoch festgestellt, daß die Hydrogenolyse auch bei Verwendung von Zink in Gegenwart von Essigsäure zufriedenstellend verläuft. Dabei ist es bevorzugt, in Gegenwart von Lösungsmitteln, wie Wasser oder Alkanolen mit 1 bis 4 Kohlenstoffatomen oder Mischungen derselben, zu arbeiten. Manchmal ist es notwendig, eine katalytische Menge einer starken Mineralsäure, wie Salzsäure, Schwefelsäure oder p-Toluolsulfonsäure, zuzugeben. Die Hydrierungsumsetzung wird zweckmäßig bei Temperaturen von etwa 40 bis 75° C durchgeführt. Als Reaktionsdauer werden vorteilhaft etwa 4 bis 7 Stunden gewählt.

Zweckmäßig werden bei der Umsetzung der Cysteinderivate der allgemeinen Formel V mit den Trialkylsilylhalogeniden der allgemeinen Formel VI der Variante c) des erfindungsgemäßen Verfahrens als letztere Trialkylsilylchloride verwendet. Die Umsetzung mit diesen Verbindungen dient dem Schutz der Carboxylgruppe. Diese Umsetzung wird vorzugsweise bei Temperaturen von etwa 5° C bis zum Siedepunkt der Reaktionsmischung durchgeführt. Als inerte organische Lösungsmittel werden für diese Umsetzung vorteilhaft niedere Halogenkohlenwasserstoffe eingesetzt. Die anschließende Acylierung des Schwefelatomes durch Umsetzen der erhaltenen Trialkylsilylcysteinderivate der allgemeinen Formel VII mit den 2-(6'-Methoxynaphth-2'-yl)-propionylhalogeniden oder 2-(2'-Äthyl-2',3'-dihydrobenzofuran-5'-yl)-acetylhalogeniden kann nach bekannten Verfahrensweisen durchgeführt werden.

Zweckmäßig wird die Variante c) des erfindungsgemäßen Verfahrens 1stufig ohne Isolierung von Zwischenproduktverbindungen wie folgt durchgeführt.

1 Moläquivalent des Cysteinderivates der allgemeinen Formel V wird in einem inerten organischen Lösungsmittel, vorteilhaft einem niederen Halogenkohlenwasserstoff, in Gegenwart von mindestens 2 Moläquivalenten einer tertiären Stickstoff aufweisenden organischen Base, beispielsweise von Pyridin oder Triäthylamin, gelöst. Die erhaltene Mischung wird bei einer Temperatur von etwa 5° C bis zum Siedepunkt der Reaktionsmischung mit etwa 1 bis 3 Moläquivalenten eines Trialkylsilylhalogenids der allgemeinen Formel VI, bezogen auf das Cysteinderivat der allgemeinen Formel V, versetzt. Diese Zugabe nimmt etwa 1 Stunde in Anspruch, worauf die erhaltene Mischung noch 1 Stunde bei Raumtemperatur stehengelassen wird, sie kann aber gegebenenfalls auch noch 1 bis 3 Stunden unter Rückfluß zum Sieden erhitzt werden. Nach der Zugabe einer etwa äquimolaren Menge von 2-(6'-Methoxynaphth-2'-yl)-propionylhalogeniden oder 2-(2'-Äthyl-2',3'-dihydrobenzofuran-5'-yl)-acetylhalogeniden, bezogen auf das betreffende eingesetzte Cysteinderivat der allgemeinen Formel V, und Halten der Reaktionsmischung während etwa 2 bis 3 Stunden auf einer Temperatur von —10° C bis Raumtemperatur wird die freie Carboxylgruppe durch Entfernen der Trialkylsilylschutzgruppe durch einfache Behandlung mit Wasser wiederhergestellt.

Die Überführung der erhaltenen erfindungsgemäßen Cysteinderivate der allgemeinen Formel I in ihre Salze kann durch übliche Verfahrensweisen erfolgen.

Es ist klar, daß bei Verwendung von 2-(6'-Methoxynaphth-2'-yl)-propionylhalogeniden der allgemeinen Formel

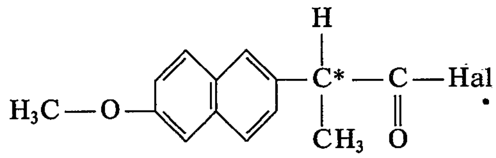

(IX)

worin Hal für Halogen steht, als Acylierungsmittel diese wegen des Vorliegens des mit dem Stern markierten asymmetrischen Kohlenstoffatomes sowohl als Racemate als auch als optisch aktive Isomere eingesetzt werden können.

Auch die Ausgangscystein- und Ausgangscystinderivate der allgemeinen Formeln II, III beziehungsweise V haben asymmetrische Kohlenstoffatome, und zwar diejenigen, an welchen die Aminogruppen hängen, und daher können nach dem erfindungsgemäßen Verfahren je nach den verwendeten Ausgangssubstanzen entweder optisch aktive Cysteinderivate der allgemeinen Formel I beziehungsweise ihre Salze oder Paare von diastereomeren Cysteinderivaten der allgemeinen Formel I beziehungsweise deren Salzen erhalten werden.

Wenn Paare von diastereomeren Cysteinderivaten der allgemeinen Formel I beziehungsweise ihrer Salze erhalten werden, können diese wie bereits erwähnt in an sich bekannter Weise in die optisch aktiven Isomere gespalten werden. Es ist jedoch bevorzugt, die Cystein- beziehungsweise Cystinderivate der allgemeinen Formeln II, III beziehungsweise V jeweils als eines der 2 möglichen optisch aktiven Isomere einzusetzen, wenn die Herstellung von optisch aktiven Cysteinderivaten der allgemeinen Formel I beziehungsweise deren Salzen gewünscht wird.

Die erhaltenen erfindungsgemäßem Verbindungen können aus den jeweiligen Reaktionsmedien durch an sich bekannte Verfahrensweisen gewonnen werden. Diese umfassen das Eindampfen der Reaktionsmischung zur Trockne beziehungsweise das Extrahieren mit Lösungsmitteln und anschließende Abdampfen der Lösungsmittel. Die erhaltenen Rückstände können durch Kristallisieren aus Lösungsmitteln oder Säulenchromatographie an Silicagel weiter gereinigt werden.

Ferner sind erfindungsgemäß Arzneimittel, welche 1 oder mehr der erfindungsgemäßen Verbindungen als Wirkstoff beziehungsweise Wirkstoffe, zweckmäßigerweise zusammen mit 1 oder mehr üblichen festen oder flüssigen pharmazeutischen Trägern und/oder Hilfsstoffen, enthalten, vorgesehen. Die erfindungsgemäßen Verbindungen haben nämlich wie bereits erwähnt wertvolle pharmakologische, insbesondere entzündungshemmende, schmerzlindernde, fiebersenkende und schleimlösende, Wirkungen.

Dabei haben sie eine niedrige Toxizität; so waren die nach der Verfahrensweise von Spearman und Karber, D. J. Finney, Statistical Methods in Biological Assays 1964, Seite 254 ermittelten peroralen $LD_{50}$-Werte an Ratten stets höher als 400 mg/kg. Eine andere vorteilhafte Eigenschaft liegt darin, daß sie nur eine geringe Neigung zur Magengeschwürbildung haben.

Die entzündungshemmende Wirkung wurde an Hand des Versuches mit dem von Carraghenin hervorgerufenen beziehungsweise induzierten Ödem (Carraghenin-Ödem) an Ratten, welcher im wesentlichen nach der Verfahrensweise von C. A. Winter und Mitarbeitern, J. Pharm. Expt. Ther. 369 [1963], 141 durchgeführt wurde, bewertet. Die Ergebnisse dieser Versuche sind in der folgenden Tabelle I zusammengestellt.

Tabelle I

| Verbindung Beispiel | Bezeichnung | Perorale Dosis in mg/kg | Hemmung des Carraghenin-Ödemes in %, bezogen auf die Blindversuche |
|---|---|---|---|
| 2 | N-[D-2-(6′-Methoxynaphth-2′-yl)-propionyl]-L-cysteinmethylester | 50 | −57 |
| 3 | N-[2-(2′-Äthyl-2′,3′-dihydrobenzofuran-5′-yl)-acetyl]-L-cystein | 50 | −48 |
| 6 beziehungsweise 7 | N-Acetyl-S-[D-2-(6′-methoxynaphth-2′-yl)-propionyl]-L-cystein | 3 | −42 |
| | | 6 | −42 |
| | | 12 | −45 |
| | | 24 | −58 |

Aus der obigen Tabelle I geht hervor, daß die erfindungsgemäßen Verbindungen bei peroraler Verabreichung in Dosen von 50 mg/kg gegenüber den Blindversuchen eine mehr als 40%ige Hemmung

des Carraghenin-Ödemwachstumes herbeiführten.

Ein weiterer Nachweis der starken entzündungshemmenden Wirkung der erfindungsgemäßen Verbindungen erfolgte mit Hilfe des Granulomwattekügelchenversuches, welcher im wesentlichen wie von R. Meier und Mitarbeitern, Experientia, 6 [1950], 1469 beschrieben durchgeführt wurde. Bei diesem Versuch wurden Granuloma durch Einsetzen eines Wattekügelchens in den intraskapularen Bereich des Versuchtieres gebildet. Die Versuchsergebnisse sind in der folgenden Tabelle II zusammengestellt.

Tabelle II

| Verbindung Beispiel | Bezeichnung | Perorale Dosis in mg/kg | Hemmung des Granulomes in %, bezogen auf die Blindversuche |
|---|---|---|---|
| 3 | N-[2-(2'-Äthyl-2',3'-dihydrobenzofuran-5'-yl)-acetyl]-L-cystein | 40 | −30 |
| 4 | N-[D-2-(6'-Methoxynaphth-2'-yl)-propionyl]-L-cystein | 40<br>80 | −24<br>−35 |
| 6 beziehungsweise 7 | N-Acetyl-S-[D-2-(6'-methoxynaphth-2'-yl)-propionyl]-L-cystein | 40<br>80 | −28<br>−49 |

Aus der obigen Tabelle II geht hervor, daß die erfindungsgemäßen Verbindungen gegenüber den Blindversuchen eine mindestens etwa 30%ige Verminderung der Granulome herbeiführten.

Es ist zu bemerken, daß die obigen 2 pharmakologischen Versuche zu 2 verschiedenen Arten von Entzündungsvorgängen in Beziehung stehen. Die Wirksamkeit beim Versuch mit dem durch Carraghenin hervorgerufenen Ödem bezieht sich auf die Verwendung der Substanz bei der Behandlung von akuten Entzündungen, während die Wirksamkeit beim Granulomwattekügelchenversuch der Behandlung von chronischen Entzündungen zuzuordnen ist.

Die schmerzlindernde Wirksamkeit wurde mit Hilfe des »Schmerzkrümmungsversuches« (»writhing test«) an Mäusen im wesentlichen nach der von Wilkin und Mitarbeitern in J. Pharm. Expt. Ther., 133 [1961], 409 beschriebenen Versuchsverfahrensweise bewertet. Repräsentative Versuche zeigten, daß die erfindungsgemäßen Verbindungen bei ihrer Verarbreichung in peroralen Dosen von etwa 30 bis 100 mg/kg gegenüber den Blindversuchen eine 50%ige Hemmung der durch das Reizmittel hervorgerufenen Unterleibsschmerzkrümmungen herbeiführten. Bei diesem Versuch zeigte sich, daß die schmerzlindernde Wirkung der erfindungsgemäßen Verbindung N-Acetyl-S-[D-2-(6'-methoxynaphth-2'-yl)-propionyl]-L-cystein (Verbindung der Beispiele 6 und 7) selbst 4 Stunden nach ihrer Verabreichung beträchtlich war. Die $ED_{50}$-Werte dieser Substanz, das heißt die Dosen, welche eine 50%ige Verminderung der Zusammenziehung der Laboratoriumstiere herbeizuführen vermögen, betrugen 25 mg/kg nach 30 Minuten und 10 mg/kg nach 4 Stunden.

Die fiebersenkende Wirkung wurde an Hamstern (Stamm Neuseeland) mit Gewichten von etwa 2 kg nach dem Erregen beziehungsweise Induzieren von Fieber beziehungsweise Hyperthermie durch intravenöses Einspritzen von Hefe in die Randvene des Ohres durch Messen der Körpertemperatur im After mittels eines Thermoelementes in vorher bestimmten Zeitbabständen untersucht. Die zu untersuchenden Verbindungen wurden 1 Stunde nach der Verabreichung des fiebererzeugenden Mittels mit einer Magensonde verabreicht. Die erhaltenen Ergebnisse zeigten, daß bei Verabreichung der erfindungsgemäßen Verbindungen in Dosen von etwa 10 bis 40 mg/kg die Körpertemperatur der Versuchstiere auf normale Werte vermindert wurde.

Die schleimlösende Wirkung wurde durch Beobachten des Verflüssigungsvermögens der erfindungsgemäßen Verbindungen bei Zugabe zu einem Probesubstrat von 1%igem Magenmucin NP-50 in einer 0,9%igen physiologischen Natriumchloridlösung bei einer Konzentration von 0,05 $Mol/cm^3$ Substrat und quantitative Bestimmung dieses Verflüssigungsvermögens mit einem Viskosimeter nach Cannon-Fenske untersucht.

Die erhaltenen Ergebnisse wurden als prozentuale Verminderung der Viskosität gegenüber den Blindversuchsproben, das heißt den Substraten, denen keine erfindungsgemäßen Verbindungen zugesetzt wurden, ausgedrückt. Es ergab sich, daß die erfindungsgemäßen Verbindungen in den oben angegebenen Konzentrationen eine beträchtliche Verminderung der Viskosität von etwa 35 bis 55% gegenüber den Blindversuchsproben herbeiführten.

Schließlich wurden die magengeschwürbildenden Eigenschaften nach der von Lumachi und Mitarbeitern in Arch. Int. Pharmacodyn., 186 [1970], 66 beschriebenen Verfahrensweise, welche im wesentlichen die Analyse der Zahl und Art der durch die zu untersuchenden Verbindungen herbeigeführten Magengeschwüre vorsieht, bewertet. Die Zahl der Tiere, welche Magenschädigungen zeigten, wurde zum Logarithmus der verabreichten Dosis in Beziehung gesetzt und so wurden die $UD_{50}$-Werte (Dosis der betreffenden Verbindung, welche bei 50% der behandelten Tiere Geschwüre herbeiführt) nach Spearman und Karber (siehe weiter oben) bestimmt. Es wurde festgestellt, daß diese $UD_{50}$-Werte um vieles höher waren als die Dosen, bei welchen die erfindungsgemäßen Verbindungen ihre vorteilhaften pharmakologischen Wirkungen entfalten.

Die erfindungsgemäßen Verbindungen können in Form von Arzneimittelpräparaten vorliegen und therapeutisch angewandt werden.

Die erfindungsgemäßen Verbindungen beziehungsweise Arzneimittelpräparate können auf verschiedenen Wegen, beispielsweise peroral, durch Inhalationen, rektal, intravenös oder intramuskulär, verabreicht werden, wobei die perorale und rektale Verabreichung bevorzugt sind. So können die erfindungsgemäßen Arzneimittelpräparate zur peroralen Verabreichung in Form von Tabletten, dispergierbaren Pulvern, Kapseln, mit Zucker überzogenen Tabletten, Körnern beziehungsweise Granulaten, Sirupen, Elixieren, Lösungen oder Aerosolen vorliegen. Die Arzneimittelpräparate zur peroralen Verabreichung können 1 oder mehr übliche Hilfsstoffe, beispielsweise Süßungsmittel, Schmackhaftmachungsmittel beziehungsweise Aromastoffe, Farbstoffe, Überzugsmittel und Konservierungsmittel, enthalten, um so ein gefälliges und schmackhaftes Arzneimittelpräparat zu haben. Die Tabletten können den erfindungsgemäßen Wirkstoff beziehungsweise die erfindungsgemäßen Wirkstoffe in Mischung mit herkömmlichen pharmazeutischen Trägern beziehungsweise Excipienten, beispielsweise inerten Verdünnungsmitteln, wie Calciumcarbonat, Natriumcarbonat, Milchzucker und/oder Talk, Granuliermitteln und/oder das Zerfallen fördernden Mitteln, wie Stärke, Alginsäure und/oder Natriumcarboxymethylcellulose, Bindemitteln, wie Stärke, Gelatine, Gummi arabicum und/oder Polyvinylpyrrolidon, und/oder Gleitmitteln, wie Magnesiumstearat, Stearinsäure und/oder Talk, enthalten. Gegebenenfalls können die Tabletten in an sich bekannter Weise überzogen sein, um ein verzögertes Zerfallen und eine verzögerte Adsorption im Magen/Darm-Trakt und damit »Retard«-Präparate zu haben.

Auch die Sirupe, Elixiere und Lösungen können in an sich bekannter Weise hergestellt sein. Sie können zusammen mit dem erfindungsgemäßen Wirkstoff beziehungsweise den erfindungsgemäßen Wirkstoffen Suspendiermittel, wie Methylcellulose, Hydroxyäthylcellulose, Traganthgummi und/oder Natriumalginat, und/oder Netzmittel, wie Lecithin, Polyoxyäthylenstearate und/oder Polyoxyäthylensorbitanmonooleat, und/oder übliche Konservierungsmittel, Süßungsmittel und/oder Puffermittel enthalten.

Die als Zäpfchen vorliegenden erfindungsgemäßen Arzneimittelpräparate können den erfindungsgemäßen Wirkstoff beziehungsweise die erfindungsgemäßen Wirkstoffe in Mischung mit Glyceriden von gesättigten Fettsäuren und/oder Polyäthylenglykolen und/oder den üblichen Konservierungsmitteln und/oder Stabilisiermitteln enthalten.

Die Wirkstoffdosis zur Bekämpfung von Entzündungs-, Schmerz- und Fieberzuständen und mit wirksamer schleimlösender Wirkung kann innerhalb weiter Grenzen je nach dem erfindungsgemäßen Wirkstoff variieren. Im allgemeinen werden gute Ergebnisse mit täglichen Dosen der erfindungsgemäßen Verbindungen von etwa 8 bis 40 mg/kg Körpergewicht erzielt.

Die Erfindungsgemäßen Arzneimittelpräparate enthalten den erfindungsgemäßen Wirkstoff beziehungsweise die erfindungsgemäßen Wirkstoffe zweckmäßig in Mengen von etwa 250 bis 1000 mg. Sie eignen sich für tägliche Einzelverabreichungen oder mehrfache Verabreichungen.

Die Erfindung wird an Hand der folgenden Beispiele näher erläutert.

## Beispiel 1

### N-[2-(2'-Äthyl-2',3'-dihydrobenzofuran-5'-yl)-acetyl]-L-cysteinmethylester

Es wurden 9,53 g (0,07 Mol) Natriumacetattrihydrat und 5,15 g (0,03 Mol) L-Cysteinmethylesterhydrochlorid in 27 $cm^3$ Tetrahydrofuran und 3 $cm^3$ Wasser in einer Stickstoffatmosphäre suspendiert. Nach dem Kühlen in einem Eisbad wurde der Reaktionsmischung eine Lösung von 7,86 g (0,045 Mol) 2-(2'-Äthyl-2',3'-dihydrobenzofuran-5'-yl)-acetylchlorid in 40 $cm^3$ Tetrahydrofuran zugetropft. Nach etwa 3 Stunden langem Stehenlassen bei Raumtemperatur bildete sich ein anorganischer Niederschlag, welcher abfiltriert und verworfen wurde; das Filtrat wurde durch Abdampfen des Lösungsmittels zur Trockne eingedampft und der erhaltene Rückstand wurde in Chloroform gelöst. Die erhaltene Lösung wurde 2mal mit einer 5%igen wäßrigen Natriumbicarbonatlösung und dann 2mal mit Wasser gewaschen. Nach dem Trocknen über Natriumsulfat und Abdampfen des Lösungsmittels wurde ein öliger Rückstand erhalten, welcher aus Benzol kristallisiert wurde.

So wurden 7,00 g (72,1% der Theorie) N-[2-(2'-Äthyl-2',3'-dihydrobenzofuran-5'-yl)-acetyl]-L-cysteinmethylester mit einem Schmelzpunkt von 84 bis 86° C und einem $[\alpha]_D^{20}$-Wert von −24,8° (c = 1,007%; Methanol) erhalten.

Beispiel 2

N-[D-2-(6'-Methoxynaphth-2'-yl)-propionyl]-L-cysteinmethylester

Diese Verbindung wurde im wesentlichen nach der im Beispiel 1 beschriebenen Verfahrensweise ausgehend von L-Cysteinmethylesterhydrochlorid und D-2-(6'-Methoxynaphth-2'-yl)-propionylchlorid in einer Ausbeute von 44,1% der Theorie mit einem Schmelzpunkt von 108 bis 109° C (aus einem Gemisch von Benzol und Cyclohexan) und einem $[\alpha]_D^{20}$-Wert von +7,5° (c = 1,00152%; Chloroform) erhalten.

Beispiel 3

N-[2-(2'-Äthyl-2',3'-dihydrobenzofuran-5'-yl)-acetyl]-L-cystein

Stufe 1

Es wurden 12,01 g (0,05 Mol) L-Cystein in 50 $cm^3$ einer 2 n Natriumhydroxydlösung gelöst und mit einer wäßrigen Lösung von 12,72 g (0,12 Mol) Natriumcarbonat in 300 $cm^3$ Wasser verdünnt und die erhaltene Lösung wurde mit 80 $cm^3$ Toluol versetzt. Nach dem Kühlen in einem Eisbad wurde während 3 Stunden eine Lösung von 24,72 g (0,11 Mol) 2-(2'-Äthyl-2',3'-dihydrobenzofuran-5'-yl)-acetylchlorid in 140 $cm^3$ Toluol zugetropft und nach dem Entfernen des Eisbades wurde die erhaltene Mischung 3 Stunden lang bei Raumtemperatur gerührt beziehungsweise geschüttelt und die beiden Phasen wurden voneinander getrennt. Die wäßrige Phase wurde mit einer 5 n Salzsäure auf einen pH-Wert von 2 gebracht und anschließend 3mal mit Äthylacetat extrahiert. Die 3 organischen Auszüge wurden vereinigt und die erhaltene Lösung wurde 3mal mit Wasser gewaschen, über Natriumsulfat wasserfrei gemacht und zur Trockne eingedampft.

So wurden 26,0 g (83,3% der Theorie) $N^1,N^2$-bis-[2-(2'-Äthyl-2',3'-dihydrobenzofuran-2'-yl)-acetyl]-L-cystin mit einem Schmelzpunkt von 94 bis 96° C (aus einem Gemisch von Isopropanol und Diisopropyläther) erhalten.

Stufe 2

Das wie vorstehend beschrieben erhaltene $N^1,N^2$-bis-[2-(2'-Äthyl-2',3'-dihydrobenzofuran-5'-yl)-acetyl]-L-cystin wurde in einer Mischung aus 90 $cm^3$ Äthanol, 30 $cm^3$ Essigsäure und 100 $cm^3$ Wasser gelöst. Die erhaltene Lösung wurde auf etwa 60° C erwärmt und mit 6,5 g Zinkstaub in kleinen Anteilen versetzt. Die Reaktionsmischung wurde etwa 7 Stunden lang auf dieser Temperatur gehalten, wobei in der 6ten Stunde 30 $cm^3$ einer 3 n Salzsäure zugesetzt wurden. Die warme Reaktionsmischung wurde filtriert, das Filtrat wurde 3mal mit Äthylacetat extrahiert und die vereinigten organischen Phasen wurden 2mal mit Wasser gewaschen. Nach dem Trocknen über Natriumsulfat wurde das Äthylacetat abgedampft und es wurde ein öliger Rückstand erhalten, welcher aus einem Gemisch von Diäthyläther und Cyclohexan kristallisiert wurde.

So wurden 19,42 g (74,4% der Theorie) N-[2-(2'-Äthyl-2',3'-dihydrobenzofuran-5'-yl)-acetyl]-L-cystein mit einem Schmelzpunkt von 92 bis 94° C und einem $[\alpha]_D^{20}$-Wert von −14,96° (c = 1,0022%; Methanol) erhalten.

Beispiel 4

N-[D-2-(6'-Methoxynaphth-2'-yl)-propionyl]-L-cystein

Diese Verbindung wurde im wesentlichen wie im Beispiel 3 beschrieben ausgehend von L-Cystin und D-2-(6'-Methoxynaphth-2'-yl)-propionylchlorid in einer Ausbeute von 68,1% der Theorie mit einem Schmelzpunkt von 142 bis 144° C (aus einem Gemisch von Diäthyläther und Cyclohexan) und einem $[\alpha]_D^{20}$-Wert von −9,96° (c = 1,00364%; Methanol) hergestellt.

Beispiel 5

N-Acetyl-S-[2-(2'-Äthyl-2',3'-dihydrobenzofuran-5'-yl)-acetyl]-L-cystein

Es wurden 9,79 g (0,06 Mol) N-Acetyl-L-cystein in einem Gemisch von 50 $cm^3$ Dimethylformamid und 12 $cm^3$ Pyridin in einer Stickstoffatmosphäre bei einer Temperatur von etwa −5° C gelöst und der erhaltenen Lösung wurden während 1 Stunde 13,48 g (0,06 Mol) 2-(2'-Äthyl-2',3'-dihydrobenzofuran-5'-yl)-acetylchlorid zugetropft. Die Reaktionsmischung wurde $1\frac{1}{2}$ Stunden lang stehengelassen

und anschließend mit 150 $cm^3$ Wasser und danach mit einer 5 n Salzsäure bis zur Erreichung eines pH-Wertes von 3,5 versetzt. Nach dem Extrahieren mit Chloroform wurden die vereinigten organischen Phasen 4mal mit Wasser gewaschen, über Natriumsulfat wasserfrei gemacht und unter Vakuum zur Trockne eingedampft. Der erhaltene Rückstand wurde an Silicagel chromatographiert, wobei zunächst mit Chloroform und dann mit einer Mischung aus Chloroform und Methanol im Volumverhältnis von 97 : 3 eluiert wurde. Die das Produkt enthaltenden Fraktionen wurden abgesondert und zur Trockne eingedampft und der erhaltene Rückstand wurde aus einem Gemisch aus Äthylacetat und Cyclohexan kristallisiert.

So wurden 10,65 g (50,5% der Theorie) N-Acetyl-S-[2-(2'-Äthyl-2',3'-dihydrobenzofuran-5'-yl)-acetyl]-L-cystein mit einem Schmelzpunkt von 98 bis 100° C erhalten.

Beispiel 6

N-Acetyl-S-[D-2-(6'-methoxynaphth-2'-yl)-propionyl]-L-cystein

Diese Verbindung wurde im wesentlichen wie im Beispiel 5 beschrieben ausgehend von N-Acetyl-L-cystein und D-2-(6'-Methoxynaphth-2'-yl)-propionylchlorid in einer Ausbeute von 33,7% der Theorie mit einem Schmelzpunkt von 123 bis 125° C (aus Äthylacetat) und einem $[\alpha]_D^{20}$-Wert von +100,6° (c = 1,01868; Chloroform) hergestellt.

Beispiel 7

N-Acetyl-S-[D-2-(6'-methoxynaphth-2'-yl)-propionyl]-L-cystein

Es wurde einer Lösung von 32,64 g (0,2 Mol) N-Acetyl-L-cystein in 250 $cm^3$ Methylenchlorid und 66,8 $cm^3$ (0,48 Mol) Triäthylamin eine Lösung von 60,8 $cm^3$ (0,48 Mol) Trimethylchlorsilan in 50 $cm^3$ Methylenchlorid während 1 Stunde zugetropft. Nach 1 Stunde langem Erhitzen zum Sieden unter Rückfluß und unter Rühren beziehungsweise Schütteln wurde eine Lösung von 44,77 g (0,18 Mol) D-2-(6'-Methoxynaphth-2'-yl)-propionylchlorid in 200 $cm^3$ Methylenchlorid während 1 Stunde zugesetzt. Das Rühren beziehungsweise Schütteln wurde etwa 2 Stunden fortgesetzt, dann wurden 200 $cm^3$ Wasser zugesetzt und die 2 Phasen wurden voneinander getrennt. Die organische Schicht wurde 2mal mit je 100 $cm^3$ Wasser gewaschen, über Natriumsulfat getrocknet und unter vermindertem Druck auf ein Volumen von 200 $cm^3$ eingeengt. Nach der Zugabe von 100 $cm^3$ Cyclohexan wurde ein Niederschlag erhalten, welcher aus Äthylacetat kristallisiert wurde.

So wurden 36,97 g (54,7% der Theorie) N-Acetyl-S-[D-2-(6'-methoxynaphth-2'-yl)-propionyl]-L-cystein mit einem Schmelzpunkt von 123 bis 125° C und einem $[\alpha]_D^{20}$-Wert von +100,6° (c = 1,01868; Chloroform) erhalten.

Beispiel 8

Es wurde in an sich bekannter Weise eine mit Zucker überzogene Tablette aus den folgenden Bestandteilen hergestellt:

| | |
|---|---|
| 600 mg | N-Acetyl-S-[D-2-(6'-methoxynaphth-2'-yl)-propionyl]-L-cystein |
| 15 mg | Natriumcarboxymethylcellulose |
| 15 mg | Magnesiumstearat |
| 20 mg | Gelatine und |
| 45 mg | Rohrzucker |
| | sowie |
| | Gummi arabicum, Milchzucker, Titandioxyd und Aluminiumlack |

Beispiel 9

Es wurde eine Kapsel aus den folgenden Bestandteilen hergestellt:

| | |
|---|---|
| 300 mg | N-Acetyl-S-[D-2-(6'-methoxynaphth-2'-yl)-propionyl]-L-cystein |
| 30 mg | Talk |
| 30 mg | Milchzucker |
| 30 mg | Natriumcarboxymethylcellulose |
| | zum Auffüllen auf |
| 500 mg | Stärke |

Beispiel 10

Es wurde eine Tablette aus den folgenden Bestandteilen hergestellt:

| | |
|---|---|
| 300 mg | N-Acetyl-S-[D-2-(6-methoxy-2-naphthyl)-propionyl]-L-cystein |
| 80 mg | Levilite® [Mischung von wasserfreien Silikaten] |
| 80 mg | Stärke |
| 40 mg | Magnesiumstearat |

Die Ausgangsverbindungen L-Cystein und L-Cystin sind Handelsprodukte.

Die Ausgangsverbindung D-2-(6'-Methoxynaphth-2'-yl)-propionylchlorid ist nach bekannten Verfahrensweisen aus der entsprechenden Säure D-2-(6'-Methoxynaphth-2'-yl)-propionsäure, welche handelsüblich ist, hergestellt worden.

Die Ausgangsverbindung 2-(2'-Äthyl-2',3'-dihydrobenzofuran-5'-yl)-acetylchlorid ist nach bekannten Verfahrensweisen aus der entsprechenden Säure 2-(2'-Äthyl-2',3'-dihydrobenzofuran-5'-yl)-essigsäure, welche in der britischen Patentschrift 1 483 061 beschrieben ist, erhalten worden.

## Patentansprüche

1. Cysteinderivate der allgemeinen Formel

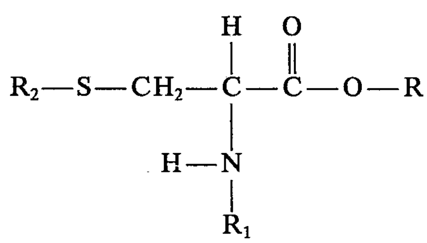

(I)

worin

R für Wasserstoff, einen Alkylrest mit 1 bis 4 Kohlenstoffatom(en), ein Alkali- oder Erdalkalimetall oder eine quaternären Ammoniumgruppe steht,

$R_1$ einen Alkylcarbonylrest mit 2 bis 4 Kohlenstoffatomen, einen 2-(6'-Methoxynaphth-2'-yl)-propionylrest oder einen 2-(2'-Äthyl-2',3'-dihydrobenzofuran-5'-yl)-acetylrest bedeutet und

$R_2$ Wasserstoff, einen 2-(6'-Methoxynaphth-2'-yl)-propionylrest oder einen 2-(2'-Äthyl-2',3'-dihydrobenzofuran-5'-yl)-acetylrest darstellt,

mit den weiteren Maßgaben, daß

a) im Falle, daß
eines von $R_1$ und $R_2$ für eine 2-(6'-Methoxynaphth-2'-yl)-propionylrest oder einen 2-(2'-Äthyl-2',3'-dihydrobenzofuran-5'-yl)-acetylrest steht,
das andere von $R_1$ und $R_2$ von einem 2-(6'-Methoxynaphth-2'-yl)-propionylrest und 2-(2'-Äthyl-2',3'-dihydrobenzofuran-5'-yl)-acetylrest verschieden ist, und

b) im Falle, daß
$R_1$ für einen Alkylcarbonylrest mit 2 bis 4 Kohlenstoffatomen steht,
$R_2$ von Wasserstoff verschieden ist,

in Form der getrennten optisch aktiven Isomere oder von Mischungen derselben.

2. Cysteinderivate nach Anspruch 1, dadurch gekennzeichnet, daß der Alkylrest, für den R stehen kann, ein solcher mit 1 oder 2, insbesondere 1, Kohlenstoffatom(en) ist.

3. Cysteinderivate nach Anspruch 1 oder 2, dadurch gekennzeichnet, daß der Alkylcarbonylrest, für den $R_1$ stehen kann, ein solcher mit 2 oder 3, insbesondere 2, Kohlenstoffatomen ist.

4. N-Acetyl-S-[2-(6'-methoxynaphth-2'-yl)-propionyl]-cystein.

5. Verfahren zur Herstellung der Verbindungen nach Anspruch 1 bis 4, dadurch gekennzeichnet, daß man in an sich bekannter Weise

a) 1 Moläquivalent Cysteinderivate der allgemeinen Formel

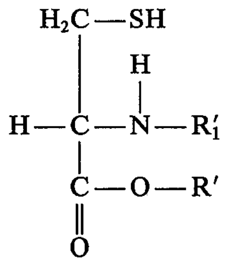 (II)

worin

R' für Wasserstoff oder einen Alkylrest mit 1 bis 4 Kohlenstoffatomen steht und
$R_1'$ Wasserstoff oder einen Alkylcarbonylrest mit 2 bis 4 Kohlenstoffatomen bedeutet,

mit etwa 1 Moläquivalent oder einem geringen Überschuß von 2-(6'-Methoxynaphth-2'-yl)-propionyl- oder 2-(2'-Äthyl-2',3'-dihydrobenzofuran-5'-yl)-acetylhalogeniden in organischen Lösungsmitteln in Gegenwart von mindestens 1 Moläquivalent alkalischen Mitteln, bezogen auf das Cysteinderivat der allgemeinen Formel II, gegebenenfalls in einer Stickstoffatmosphäre, umsetzt oder

b) zur Herstellung der Verbindungen der allgemeinen Formel I, bei welchem

$R_1$ für einen 2-(6'-Methoxynaphth-2'-yl)-propionyl- oder 2-(2'-Äthyl-2',3'-dihydrobenzofuran-5'-yl)-acetylrest steht und
$R_2$ Wasserstoff bedeutet,

1 Moläquivalent Cystinderivate der allgemeinen Formel

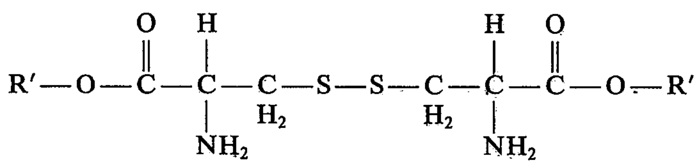 (III)

worin R' für Wasserstoff oder einen Alkylrest mit 1 bis 4 Kohlenstoffatomen steht, oder Säureadditionssalze derselben mit etwa 2 Moläquivalenten oder einem geringen Überschuß darüber von 2-(6'-Methoxynaphth-2'-yl)-propionyl- oder 2-(2'-Äthyl-2',3'-dihydrobenzofuran-5'-yl)-acetylhalogeniden in inerten organischen Lösungsmitteln in Gegenwart von alkalischen Mitteln zu $N^1$,$N^2$-bis-[2-(6'-Methoxynaphth-2'-yl)-propionyl]-cystin beziehungsweise $N^1$,$N^2$-bis-[2-(2'-Äthyl-2',3'-dihydrobenzofuran-5'-yl)-acetyl]-cystin beziehungsweise ihren Estern der allgemeinen Formel

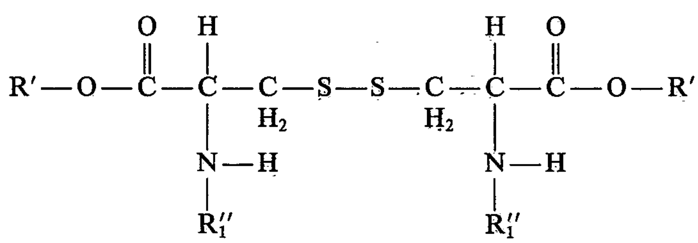 (IV)

worin

$R_1''$ für einen 2-(6'-Methoxynaphth-2'-yl)-propionyl- oder 2-(2'-Äthyl-2',3'-dihydrobenzofuran-5'-yl)-acetylrest steht und
R' wie vorstehend festgelegt ist,

umsetzt und die letzteren, gegebenenfalls ohne ihre Isolierung, katalytisch hydriert oder

c) zur Herstellung der Verbindungen der allgemeinen Formel I, bei welchem

R für Wasserstoff, ein Alkali- oder Erdalkalimetall-Äquivalent oder eine quaternäre Ammoniumgruppe steht,
$R_1$ einen Alkylcarbonylrest mit 2 bis 4 Kohlenstoffatomen bedeutet und
$R_2$ einen 2-(6'-Methoxynaphth-2'-yl)-propionyl- oder 2-(2'-Äthyl-2',3'-dihydrobenzofuran-5'-yl)-acetylrest darstellt,

1 Moläquivalent Cysteinderivate der allgemeinen Formel

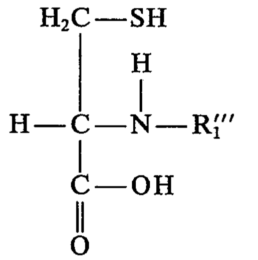

(V)

worin $R_1'''$ für einen Alkylcarbonylrest mit 2 bis 4 Kohlenstoffatomen steht, beziehungsweise Säureadditionssalze derselben, mit etwa 1 bis 3 Moläquivalenten Trialkylsilylhalogeniden der allgemeinen Formel

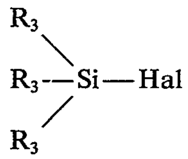

(VI)

worin

$R_3$ für einen Alkylrest mit 1 bis 4 Kohlenstoffatomen steht und
Hal Halogen bedeutet,

in inerten organischen Lösungsmitteln in Gegenwart von mindestens 2 Moläquivalenten von tertiärem Stickstoff aufweisenden organischen Basen zu geschützten Cysteinderivaten der allgemeinen Formel

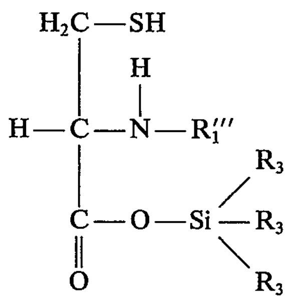

(VII)

worin $R_1'''$ und $R_3$ wie vorstehend festgelegt sind, umsetzt, diese durch Zugabe von etwa 1 Moläquivalent 2-(6'-Methoxynaphth-2'-yl)-propionyl- beziehungsweise 2-(2'-Äthyl-2',3'-dihydrobenzofuran-5'-yl)-acetylhalogeniden zu geschützten Cysteinderivaten der allgemeinen Formel

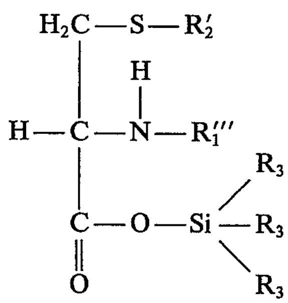

(VIII)

worin

$R_2'$ für einen 2-(6'-Methoxynaphth-2'-yl)-propionyl- oder 2-(2'-Äthyl-2',3'-dihydrobenzofuran-5'-yl)-acetylrest steht und
$R_1'''$ und $R_3$ wie vorstehend festgelegt sind,

umsetzt und die die letzteren enthaltenden Reaktionsmischungen zur Wiederherstellung der freien Carboxylgruppe mit Wasser behandelt, wobei man alle diese Umsetzungen ohne Isolierung von Zwischenprodukten durchführen kann,
worauf man in an sich bekannter Weise gegebenenfalls die gegebenenfalls erhaltenen Cysteinderivate der allgemeinen Formel I, bei welcher R für Wasserstoff steht, in ihre Alkali- oder Erdalkalimetallsalze oder quaternären Ammoniumsalze überführt und/oder gegebenenfalls die erhaltenen Mischungen der

optisch aktiven Isomere der Cysteinderivate der allgemeinen Formel I in die optisch aktiven Isomere spaltet.

6. Arzneimittel, gekennzeichnet durch einen Gehalt an 1 oder mehr Verbindungen nach Anspruch 1 bis 4, gegebenenfalls zusammen mit 1 oder mehr festen oder flüssigen pharmazeutischen Trägern und/oder Hilfsstoffen.

**Claims**

1. Cysteine derivatives having the general formula

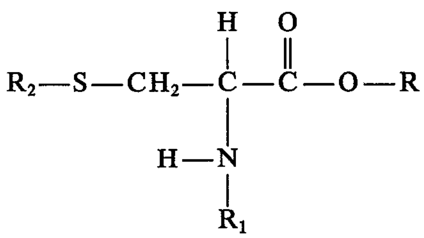

(I)

wherein

R represents hydrogen, an alkyl radical having from 1 to 4 carbon atom(s), an alkali or alkaline-earth metal or a quaternary ammonium group,

$R_1$ means an alkyl carbonyl radical having from 2 to 4 carbon atoms, a 2-(6′-methoxynapht-2′-yl)-propionyl radical or a 2-(2′-ethyl-2′,3′-dihydro benzofuran-5′-yl)-acetyl radical and

$R_2$ represents hydrogen, a 2-(6′-methoxynapht-2′-yl)-propionyl radical or a 2-(2′-ethyl-2′,3′-dihydro benzofuran-5′-yl)-acetyl radical,

with the further definitions that

a) in case that
one of $R_1$ and $R_2$ represents a 2-(6′-methoxynapht-2′-yl)-propionyl radical or a 2-(2′-ethyl-2′,3′-dihydrobenzofuran-5′-yl)-acetyl radical,
the other one of $R_1$ and $R_2$ is different from a 2-(6′-methoxynapht-2′-yl)-propionyl radical and 2-(2′-ethyl-2′,3′-dihydrobenzofuran-5′-yl)-acetyl radical and

b) in case that
$R_1$ reprensents an alkyl carbonyl radical having from 2 to 4 carbon atoms
$R_2$ is different form hydrogen

in the form of the separated optically active isomers or of mixtures of them.

2. Cysteine derivatives according to claim 1, characterized in that the alkyl radical which may be represented by R is such a one having 1 or 2, particularly 1, carbon atom(s).

3. Cysteine derivatives according to claim 1 or 2, characterized in that the alkyl carbonyl radical which may be represented by $R_1$ is such a one having 2 or 3, particularly 2, carbon atoms.

4. N-acetyl-S-[2-(6′-methoxynapht-2′-yl)-propionyl]-cysteine.

5. A process for preparing the compounds according to claims 1 to 4, characterized in that in a manner known per se

a) one reacts 1 mol equivalent of cysteine derivatives of the general formula

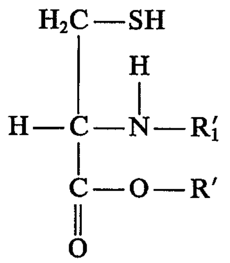

(II)

wherein

R′ represents hydrogen or an alkyl radical having from 1 to 4 carbon atom(s) and

$R'_1$ means hydrogen or an alkyl carbonyl radical having from 2 to 4 carbon atoms

with about 1 mol equivalent or a slight excess of 2-(6′-methoxynapht-2′-yl)-propionyl or 2-(2′-ethyl-2′,3′-dihydrobenzofuran-5′-yl)-acetyl halides in organic solvents in the presence of at least 1 mol equivalent of alkaline agents, referred to the cysteine derivative having the general formula II, optionally in a nitrogen atmosphere or

b) for preparing the compounds of the general formula I in which

$R_1$ represents a 2-(6'-methoxynapht-2'-yl)-propionyl or 2-(2'-ethyl-2',3'-dihydrobenzofuran-5'-yl)-acetyl radical and

$R_2$ means hydrogen

one reacts 1 mol equivalent of cystéine derivatives having the general formula

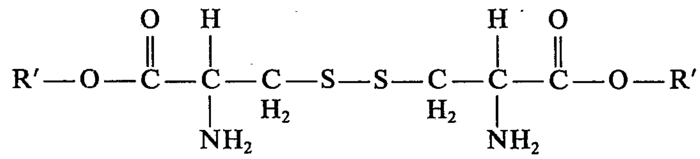

(III)

wherein R' represents hydrogen or an alkyl radical having from 1 to 4 carbon atom(s) or acid addition salts of them with about 2 mol equivalents or a slight excess over this of 2-(6'-methoxynapht-2'-yl)-propionyl or 2-(2'-ethyl-2',3'-dihydrobenzofuran-5'-yl)-acetyl halides in inert organic solvents in the presence of alkaline agents to yield $N^1,N^2$-bis-[2-(6'-methoxynapht-2'-yl)-propionyl]-cystéine or $N^1,N^2$-bis-[2-(2'-ethyl-2',3'-dihydrobenzofuran-5'-yl)-acetyl]-cystéine or their esters, respectively, having the general formula

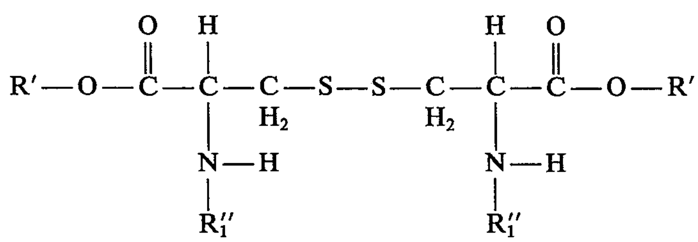

(IV)

wherein

$R_1''$ represents a 2-(6'-methoxynapht-2'-yl)-propionyl or 2-(2'-ethyl-2',3'-dihydrobenzofuran-5'-yl)-acetyl radical and

R' is as in the foregoing defined

and one hydrogenises catalytically the latter, optionally without their isolation, or

c) for preparing the compounds of the general formula I in which

R represents hydrogen, an alkaline or alkaline-earth metal equivalent or a quaternary ammonium group,

$R_1$ means an alkyl carbonyl radical having from 2 to 4 carbon atoms and

$R_2$ represents a 2-(6'-methoxynapht-2'-yl)-propionyl or 2-(2'-ethyl-2',3'-dihydrobenzofuran-5'-yl)-acetyl radical

one reacts 1 mol equivalent of cysteine derivatives having the general formula

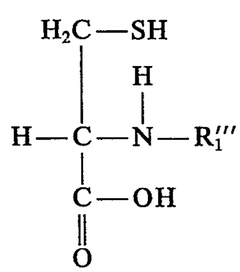

(V)

wherein $R_1'''$ represents an alkyl carbonyl radical having from 2 to 4 carbon atoms or acid addition salts of them, respectively with about 1 to 3 mol equivalents of trialkylsilyl halides having the general formula

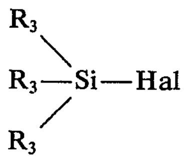

(VI)

wherein

$R_3$ represents an alkyl radical having from 1 to 4 carbon atoms and
Hal means halogen

in inert organic solvents in the presence of at least 2 mol equivalents of organic bases containing tertiary nitrogen to yield protected cysteine derivatives having the general formula

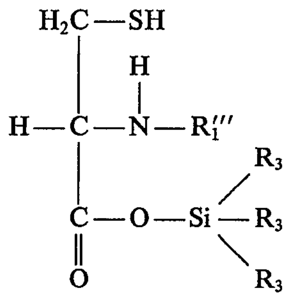

(VII)

wherein $R_1'''$ and $R_3$ are defined as in the foregoing, one reacts these by adding about 1 mol equivalent of 2-(6'-methoxynapht-2'-yl)-propionyl or 2-(2'-ethyl-2',3'-dihydrobenzofuran-5'-yl)-acetyl halides, respectively, to yield cysteine derivatives having the general formula

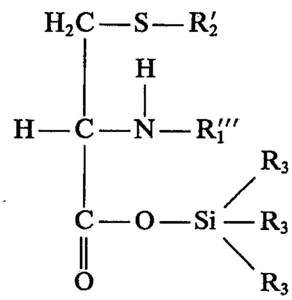

(VIII)

wherein

$R_2'$ represents a 2-(6'-methoxynapht-2'-yl)-propionyl or 2-(2'-ethyl-2',3'-dihydrobenzofuran-5'-yl)-acetyl radical and
$R_1'''$ and $R_3$ are defined as in the foregoing

and one treats the reaction mixtures containing the latter one with water for restoring the free carboxylic group being possible to carry out all these reactions without isolating intermediates thereafter in a manner known per se optionally one converts the cysteine derivatives having the general formula I in which R represents hydrogen, optionally obtained, into their alkali or alkaline-earth metal salts or quaternary ammonium salts and/or optionally one separates the mixtures of the optically active isomers of the cysteine derivates having the general formula I, optionally obtained, to the optically active isomers.

6. Medicaments, characterized by a content of 1 or more compounds according to claims 1 to 4, optionally together with 1 or more solid or liquid supports and/or auxilliary materials.

**Revendications**

1. Dérivés de cystéine de formule générale:

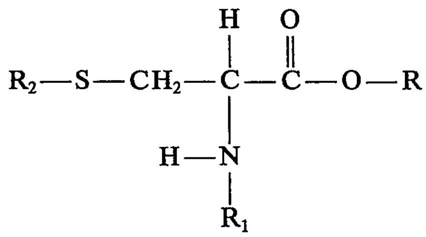

(I)

dans laquelle:

R représente l'hydrogène, un radical alkyle de 1 à 4 atomes de carbone, un métal alcalin ou alcalino-terreux ou un groupe ammonium quaternaire,
$R_1$ représente un radical alkylcarbonyle de 2 à 4 atomes de carbone, un radical 2-(6'-méthoxynapht-2'-yl)-propionyle ou un radical 2-(2'-éthyl-2',3'-dihydrobenzofurann-5'-yl)-acétyle et
$R_2$ représente l'hydrogène, un radical 2-(6'-méthoxynapht-2'-yl)-propionyle ou un radical 2-(2'-éthyl-2',3'-dihydrobenzofurann-5'-yl)-acétyle,
étant entendu en outre que:

a) dans le cas où l'un de $R_1$ ou de $R_2$ représente un radical 2-(6'-méthoxynapht-2'-yl)-propionyle ou un radical 2-(2'-éthyl-2',3'-dihydrobenzofurann-5'-yl)-acétyle, l'autre substituante ($R_1$ ou $R_2$) est différent d'un radical 2-(6'-méthoxynapht-2'-yl)-propionyle et d'un radical 2-(2'-éthyl-2',3'-dihydrobenzofurann-5'-yl)-acétyle, et que
b) dans le cas où $R_1$ représente un radical alkylcarbonyle contenant 2 à 4 atomes de carbone, $R_2$ est différent de l'hydrogène,

sous la forme des isomères optiquement actifs séparés ou de mélanges de ceux-ci.

2. Dérivés de cystéine selon la revendication 1, caractérisés en ce que le radical alkyle que peut représenter R est un radical contenant 1 ou 2 atomes de carbone, en particulier 1.

3. Dérivés de cystéine selon l'une des revendications 1 et 2, caractérisés en ce que le radical alkylcarbonyle que peut représenter $R_1$ est un radical contenant 2 ou 3 atomes de carbone, en particulier 2.

4. N-acétyl-S-[2-(6'-méthoxynapht-2'-yl)-propionyl]-cystéine.

5. Procédé de préparation des composés selon les revendications 1 à 4, caractérisé par le fait que de manière en elle-même connue:

a) on fait réagir un équivalent molaire de dérivés de cystéine de formula génerale:

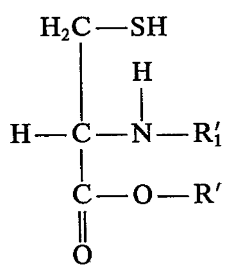

(II)

dans laquelle:

R' représente l'hydrogène ou un radical alkyle de 1 à 4 atomes de carbone et
$R'_1$ représente l'hydrogène ou un radical alkylcarbonyle de 2 à 4 atomes de carbone,

sur environ un équivalent molaire ou un léger excès d'halogénures de 2-(6'-méthoxynapht-2'-yl)-propionyle ou de 2-(2'-éthyl-2',3'-dihydrobenzofurann-5'-yl)-acétyle dans des solvants organiques en présence d'au moins un équivalent molaire d'agents alcalins par rapport au dérivé de cystéine de formule générale II, éventuellement en atmosphère d'azote, ou

b) pour la préparation des composés de formule générale I dans lesquels:

$R_1$ représente un radical 2-(6'-méthoxynapht-2'-yl)-propionyle ou 2-(2'-éthyl-2',3'-dihydrobenzofurann-5'-yl)-acétyle et
$R_2$ représente l'hydrogène,

on fait réagir un équivalent molaire de dérivés de cystéine de la formule générale:

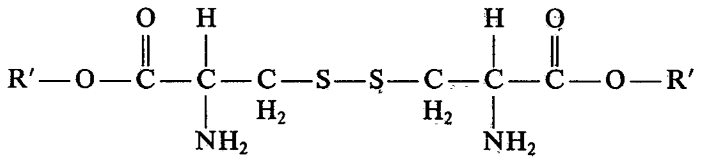

(III)

dans laquelle R' représente l'hydrogène ou un radical alkyle de 1 à 4 atomes de carbone, ou de sels d'addition d'acide de ces dérivés, sur environ deux équivalents molaires, ou un léger excès d'halogénures de 2-(6'-méthoxynapht-2'-yl)-propionyle ou de 2-(2'-éthyl-2',3'-dihydrobenzofurann-5'-yl)-acétyle dans des solvants organiques inertes en présence d'agents alcalins pour obtenir respectivement la $N^1,N^2$-bis-[2-(6'méthoxynapht-2'-yl)-propionyl]-cystéine et la $N^1,N^2$-bis-[2-(2'-éthyl-2',3'-dihydrobenzofurann-5'-yl)-acétyl]-cystéine ou leurs esters, de la formule générale:

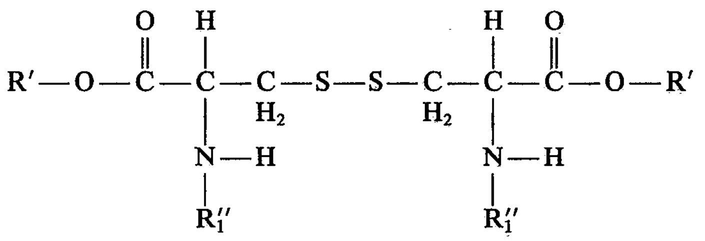

(IV)

dans laquelle:

$R_1''$ représente un radical 2-(6'-méthoxynapht-2'-yl)-propionyle ou 2-(2'-éthyl-2',3'-dihydrobenzofurann-5'-yl)acétyle et
R' est tel que défini en haut,

et que l'on hydrogène catalytiquement ces derniers éventuellement sans les isoler, ou
c) pour la préparations des composés de formule générale I dans lesquels:

R représente l'hydrogène, un équivalent de métal alcalin ou alcanilo-terreux ou un groupe ammonium quaternaire,
$R_1$ représente un radical alkylcarbonyle de 2 à 4 atomes de carbone et
$R_2$ représente un radical 2-(6'-méthoxynapht-2'-yl)-propionyle ou 2-(2'-éthyl-2',3'-dihydrobenzofurann-5'-yl)-acétyle,

on fait réagir un équivalent molaire de dérivés de cystéine de la formule générale:

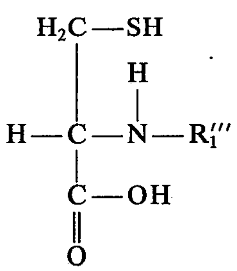

(V)

dans laquelle $R_1'''$ représente un radical alkylcarbonyle de 2 à 4 atomes de carbone, ou de sels d'addition d'acide de ces dérivés, sur environ 1 à 3 équivalents molaires d'halogénures de trialkylsilyle de la formule générale:

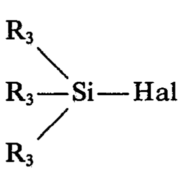

(VI)

dans laquelle:

$R_3$ représente un radical alkyle de 1 à 4 atomes de carbone et
Hal signifie halogène,

dans des solvants organiques inertes en présence d'au moins deux équivalents molaires de bases organiques comportant de l'azote tertiaire, pour obtenir des dérivés de cystéine protégés de la formule générale:

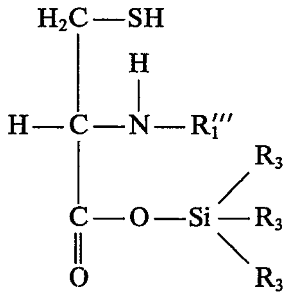

(VII)

dans laquelle $R_1'''$ et $R_3$ sont tels que définis plus haut, on convertit ceux-ci, par addition d'environ un équivalent molaire d'halogénures de 2-(6'-méthoxynapht-2'-yl)-propionyle ou de 2-(2'-éthyl-2',3'-dihydrobenzofurann-5'-yl)-acétyle, en dérivés de cystéine protégés de la formule générale:

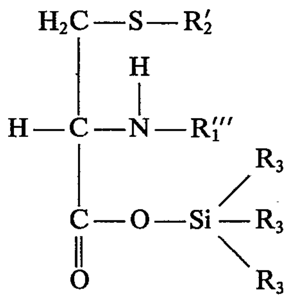

(VIII)

dans laquelle:

| | |
|---|---|
| $R_2'$ | représente un radical 2-(6'-méthoxynapht-2'-yl)-propionyle ou 2-(2'-éthyl-2',3'-dihydrobenzofurann-5'-yl)-acétyle et |
| $R_1'''$ et $R_3$ | sont tels que définis plus haut, |

et que l'on traite par l'eau les mélanges réactionnels contenant ces derniers pour rétablir le groupe carboxyle libre, toutes ces réactions pouvant être conduites sans isolement de produits intermédiaires, après quoi, de manière en elle-même connue, on convertit éventuellement les dérivés de cystéine de formule générale I dans lesquels R représente l'hydrogène, éventuellement obtenus, en leurs sels de métaux alcalins ou alcalino-terreux on en leurs sels d'ammonium quaternaire et/ou éventuellement, on dédouble les mélanges des isomères optiquement actifs des dérivés de cystéine de formule générale I, ainsi obtenus, pour obtenir les isomères optiquement actifs.

6. Médicaments caractérisés en ce qu'ils contiennent un ou plusieurs composés selon les revendications 1 à 4, éventuellement en même qu'un ou plusieurs véhicules et/ou adjuvants pharmaceutiques solides ou liquides.